# EUROPEAN PATENT SPECIFICATION

(11) **EP 1 285 654 B1**
(45) Date of publication and mention of the grant of the patent: **07.06.2006**
(21) Application number: 02705275.2
(22) Date of filing: 15.03.2002
(51) Int. Cl.: A61K 31/4172, A61K 31/195, A61K 38/05, A61P 25/02, A61P 3/10, A61P 9/12, A23L 1/305, A23L 2/52

(54) **AUTONOMIC SYSTEM CONTROLLING AGENTS AND HEALTH DRINKS AND FOODS**
AUTONOMSYSTEM KONTROLLAGENTIEN UND GESUNDHEITSGETRÄNKE UND -NAHRUNGSMITTEL
AGENTS DE REGULATION DU SYSTEME AUTONOME, BOISSONS ET ALIMENTS DIETETIQUES

(30) Priority: 16.03.2001 JP 2001076738
(43) Date of publication of application: 26.02.2003
(73) Proprietor: SUNTORY LIMITED, Osaka-shi, Osaka 530-8203 (JP); Cerebos Pacific Limited, Singapore 237994 (SG)
(72) Inventor: TSURUOKA, Nobuo, Ibaraki-shi, Osaka 567-0828 (JP); KISO, Yoshinobu, Ibaraki-shi, Osaka 567-0872 (JP); NAGAI, Katsuya, Minoo-shi, Osaka 562-0044 (JP); MATSUMURA, Yasuo, Shinjyo-cho, Kitakatsuragi-gun, Nara 639 (JP); NIJIMA, Akira, Niigata-shi, Niigata 951-8151 (JP); MORITANI, Toshio, 834 Godoshukusha, Kyoto-shi, Kyoto 612-8034 (JP)
(74) Representative: Ford, Michael Frederick
(86) International application number: PCT/JP2002/002520
(87) International publication number: WO 2002/076455

(56) References cited:
- JP-A- 11 322 617
- JP-A- 2000 198 739
- JP-A- 2001 057 869
- US-A- 5 561 110
- US-A- 5 585 396

## Description

The present invention relates to the use of carnosine in preparation of compositions suitable for the administration of carnosine at a low dose, i.e. a low-dose drug, for specified purposes.

### Background

Living organisms are equipped with the autonomic nervous system, the endocrine system, and the immune system in order to maintain homeostasis therein. Among these, the autonomic nervous system is responsible for the control of blood glucose level, blood pressure, gastric juice secretion, body temperature etc. in the living body ("The regulatory system in living organisms" in Iwanami Koza "Gendai Igaku-no Kiso (Basic Modern Medicine)" Vol. 4, edited by Toshio Hagiwara and Seiichi Tarui, 1999). The autonomic nervous system is composed of the sympathetic nerve system and the parasympathetic nerve system, and the balance of the two provides the control thereof. Thus, when the living body is in action, the activity of the sympathetic nerve system becomes dominant resulting in a systemic tension state. Conversely, when the activity of the parasympathetic nerve system is dominant, tension is relieved resulting in a relaxed state.

The activity of the sympathetic nerves, when systemically enhanced, leads to the promotion of hormone secretion, increases in heart rate and blood pressure, expansion of bronchioles, suppression of intestinal motility and secretion, enhanced glucose metabolism, dilatation of the pupil, piloerection, constriction of the skin and blood vessels in the internal organs, and further the vaspdilatation of the skeletal muscles ("The regulatory system in living organisms" in Iwanami Koza "Gendai Igaku-no Kiso (Basic Modern Medicine)" Vol. 4, edited by Toshio Hagiwara and Seiichi Tarui, 1999). Thus, when the activity of the sympathetic nerve system is enhanced, health disturbances, such as hypertension, hyperglycemia, decreased blood circulation in the skin, decreased immune functions, and the like, occur. On the other hand, when the activity of the parasympathetic nerve system is enhanced, chronic constipation may occur.

Currently, these health disturbances have been symptomatically treated depending on each of the symptoms and the disorders, and no measures have been taken to control the fundamental cause of the action of the autonomic nervous system per se. Symptomatic treatments indeed can temporalily improve the disease state but the withdrawal of medication may lead to remission, and if the medication is continued, other disorders due to the same disturbed unbalances may occur in many cases. Thus, there has been a need for pharmaceutical drugs or foods that can control the fundamental cause of the autonomic nerve activity per se.

L-carnosine (β-alanyl-L-histidine, hereinafter referred to as carnosine) is a dipeptide discovered from the skeletal muscles in 1900's, and is present in high concentrations (1-20 mM) in the skeletal muscles of various vertebrates (Nippon Rinsho 47:476-480, 1989). Carnosine is a water-soluble antioxidant in the living body (Cariballa SE and Sinclair AJ, Age Ageing, 29:207-210, 2000) as are other histidine-containing dipeptides such as anserine and homocarnosine, with the antioxidant actions of anserine and homocarnosine being more potent followed by carnosine (Kohen R et al., Proc. Natl. Acad. Sci. 85:3175-3179, 1988). Carnosine serves to stabilize the cell membrane by preventing the peroxidization of lipids in the membrane or by eliminating superoxide anions (Boldyrev AA et al., Mol. Chem. Neuropath., 19:185-192, 1993).

Carnosine is known not only as an antioxidant but as a neurotransmitter, an enzyme activity modulator, and a chelating agent for metal ions (Cariballa SE and Sinclair AJ, Age Ageing, 29:207-210, 2000). It has also been demonstrated that carnosine has physiological effect such as an anti-aging effect (Bioscience Report, 19:581-587, 1999), a wound-healing effect (Roberts PR et al., Nutrition 14:266-269, 1998), a vasodilatory effect using the isolated blood vessel (Ririe DG et al., Nutrition 16:168-172, 2000) and a cardiotonic effect (US-A-5585396). Based on these pluripotent physiological actions, carnosine has been indicated for rheumatoid and other polyarthritideson, duodenal ulcer or gastric ulcer, inflammation associated with dental extractions and parodontosis, inoperable tumors, and the like (Quinn PJ et al., Mol. Aspects Med., 13:379-444, 1992).

It has been shown (in Japanese PCT Patent publication (Kohyo) 07-500580 and US-A-5561110) that, as a method of treating complications and etiology of diabetes mellitus with carnosine, the ingestion of a 2% aqueous solution of carnosine is effective for the treatment of atherosclerosis and cataract in rabbits. The dosage of carnosine to rabbits in the Examples of that invention is 800 mg/kg body weight/day or more (oral ingestion).

It has been described (Japanese PCT Patent Publication (Kohyo) No. 11-505540) that a pharmaceutical composition and/or a nutritional composition having an antioxidant activity, said composition comprising carnosine or a derivative and a branched amino acid thereof, can prevent the formation of lipid peroxides in the internal organs by allowing a 0.75% aqueous solution of carnosine to be administered to rats. In that invention, carnosine is administered at 300 mg/kg body weight/day or more (oral ingestion).

A therapeutic agent (Pat No. 2777908) for pancreatitis having a zinc salt of carnosine as an active ingredient has been orally administered to rats at 100 mg/kg body weight/day or more.

For the effect of protecting hepatic functions and of promoting the metabolism of stress-related substances (Nippon Seirishi 52:221-228, 1990), carnosine has been administered to rats at 250 mg/kg body weight/day or more (oral ingestion), and to rats and mice at 100 mg/kg body weight/day or more (parenteral administration). Thus, the physiological effect of carnosine that has been shown in animal experiments has been confirmed when carnosine was administered at 100 mg/kg body weight/day or more.

On the other hand, it has been described that the oral administration to humans at 50 mg to 5 g/day is required for carnosine to exhibit an effect of promoting the absorption of iron (JP-A-7/097323).

Furthermore, for carnosine as a preventive and alleviating agent for medical symptoms resulting from decreased erythrocyte counts, the administration to humans at 50 mg to 5 g/day (oral administration) or 5-500 mg/day (parenteral administration) is required. Also, for the effect of carnosine on enhancing learning ability (JP-A-9/020661) the administration to humans at 50 mg to 5 g/day (oral administration) or 5-500 mg/day (parenteral administration) is required. Thus, for carnosine to exhibit its confirmed physiological effect in humans, the ingestion of 50 mg/day or more in the case of oral administration is required in either case.

Thus, although it has become clearer that carnosine has a variety of physiological activities, its mechanism of action is based on the antioxidant effect (Kohen R. et al., Proc. Natl. Acad. Sci. 85:3175-3179, 1988) or the metal chelating effect (Chem. Lett. 88:335-338, 1988), and the dosage is not small at all. Thus, according to the reports that have been made so far, the ingestion of 100 mg/kg body weight/day or more is required for carnosine to exert its physiological effect in animals, and for carnosine to exert its physiological effect in humans, the ingestion of 50 mg/day or more is required in the case of oral administration.

As hereinabove described, despite the variety of actions reported for carnosine, they have focused only on its effect in symptomatic treatments for various disease states. Furthermore a high amount of carnosine is administered, and no reports have been made that indicate that the ingestion of carnosine at a low dose may play a role in the control of the autonomic nerve activity.

Thus, in order to maintain homeostasis or to restore homeostasis that has been disturbed in the living organism, it is one aspect of the present invention to provide foods and drinks capable of being ingested at a precise amount that has the effect of acting directly on the autonomic nerves and of controlling the activity of the autonomic nerves, and methods for attaining it.

In addressing this issue, the inventors noted the chicken essence which Mr. Brand, a chef at Buckingham Palace in England more than about 160 years ago, obtained for maintaining the health of the royal family.

In recent years, scientific research has been made on the efficacy of chicken essence. An effect of enhancing basic metabolism (Nutr. Reports Int. 39:547-556, 1989) and a recovery effect from mental fatigue (Appl. Human Sci. 15:281-286, 1996) have been reported.

The chicken essence was stripped of fats by the double boiling method, in which the whole chicken was boiled twice, and contained a large amount of amino acids, peptides, and proteins. As foods and drinks rich in amino acids, peptides, and proteins, there have been known e.g. collagen peptides, soy bean peptides. Ingredients characteristic of this chicken essence include carnosine and anserine, derived from the muscles of chicken. The inventors of the present invention have studied the effect of carnosine on the autonomic nervous system.

It has been found that when carnosine was intravenously administered to rats, carnosine at low doses suppressed the neural activities of the sympathetic efferent nerves (the sympathetic nerves innervating the adrenal gland, the liver, and the kidney), while it excited the neural activity of the parasympathetic efferent nerves (the vagal nerve innervating the abdominal cavity).

If carnosine acts directly on the activity of the autonomic nervous system, the direct action of carnosine on the autonomic nerves is expected to influence the biological regulatory mechanism such as the control of blood glucose level, the control of blood pressure, the control of gastric juice secretion, the control of body temperature and the like. That is, since the direct action of carnosine on the activity of the autonomic nervous system is to suppress the activity of the sympathetic nerve system and to promote the activity of the parasympathetic nerve system, it should act in the direction of hypoglycemia in the control of blood glucose level and of hypotension in the control of blood pressure.

Also, as carnosine promoted the activity of the parasympathetic hepatic nerve (vagal nerve), it should promote glucose uptake in the liver resulting in decreased levels of blood glucose. Also, as carnosine suppressed the activity of the sympathetic hepatic nerve, it should suppress the liberation of glucose into the blood resulting in decreased levels of blood glucose. Furthermore, as carnosine suppressed the activity of the parasympathetic adrenal nerve, it should suppress the secretion of adrenaline, which should suppress the hyperglycemia.

That is, as a result of allowing carnosine at low doses to act directly on the autonomic nervous system, blood glucose levels are expected to be decreased. This was demonstrated in an experiment in which carnosine at low doses was intraperitoneally administered to the 2-deoxy-D-glucose (2DG) hyperglycemic rats, an animal model that exhibits hyperglycemia due to the enhancement of the sympathetic neural activities, to investigate the hypoglycemic action of carnosine, and the result indicated, as expected, that carnosine at low doses exhibited an effect of decreasing blood glucose levels. Carnosine at high doses does not exhibit such an activity. Thus, the controlling effect of carnosine on the autonomic nervous system was observed only when certain low levels of carnosine were ingested.

Anserine is a histidine-containing dipeptide as is carnosine, and is a methylated product of carnosine in which position 1 of the imidazole ring of carnosine has been methylated. Thus, in order to investigate whether the hypoglycemic effect of carnosine is unique to carnosine or not, the hypoglycemic effect of anserine was examined. As a result, no effect of the hypoglycemic action was noted in anserine. Thus, it was revealed that the hypoglycemic effect of carnosine at a low dose was unique to carnosine.

Then, it was investigated whether the hypoglycemic action of carnosine by controlling the autonomic nervous system was also effective in the streptozotocin (STZ) diabetic rats in which proinsulin biosynthesis has been inhibited by the destruction of beta cells in the pancreas. In the STZ diabetic rats undergoing the oral glucose tolerance test, carnosine at a low dose was confirmed to exhibit the effect of decreasing blood glucose levels. Thus, it is believed that, in the STZ diabetic rats having an insufficiency of insulin secretion, carnosine promoted glucose uptake in the liver by promoting the parasympathetic hepatic nerve (vagal nerve) activity, resulting in the hypoglycemia, and thus it was confirmed that carnosine at a low dose had an anti-diabetic effect.

Furthermore, as carnosine at a low dose suppressed the sympathetic adrenal nerve activity, adrenaline secretion will be suppressed, and increases in cardiac output by adrenaline will be suppressed, with a result that blood pressure will be lowered. Accordingly, the hypotensive effect of carnosine at a low dose was examined using deoxycorticosterone acetate (DOCA)-salt hypertensive rats, and it was confirmed that the blood pressure of the rats that fed with the diets containing carnosine at low doses was decreased.

From these animal experiments, it was thought that carnosine exerted a similar effect to the activity of the autonomic nervous system in humans. Thus, the activity of the autonomic nervous system in the heart was examined in a method (Science 213:220-222, 1981) in which the electrocardiogram (ECG) of the human heart was taken and the intervals of the R waves (R-R intervals) in the ECG were measured, followed by the spectral analysis of heart rate variation. As a result, it was seen that the oral ingestion of carnosine at low doses promoted the activity of the parasympathetic nerve system in the heart, and the overall activity of the autonomic nervous system at this time was lower than the promotion of the parasympathetic nerve activity, and hence it was found that the low doses of carnosine inhibited the activity of the sympathetic nerve system in humans as well.

From these results, the inventors of the present invention have discovered the controlling effect of carnosine on the autonomic nervous system in animal experiments, that is, carnosine at low doses suppressed the neural activities of the sympathetic efferent nerve and promoted the neural activity of the parasympathetic efferent nerve. Also, as carnosine at low doses exhibited the effect of controlling the autonomic nervous system, it was confirmed that it acted on the control of blood glucose level and blood pressure by controlling the autonomic nervous system in animal models of hyperglycemia and of hypertension. Furthermore, the inventors have found that carnosine at low doses has an effect of controlling the autonomic nervous system in humans, and thereby have completed the present invention.

Thus, a problem addressed herein is to provide new compositions having an effect of controlling the autonomic nervous system. A proposal herein for this purpose is the use of carnosine to make such a composition, in an amount to provide 0.7 µg/kg to 0.09 mg/kg of carnosine per ingestion.

In a first aspect, the present invention provides the use of carnosine in the production of a composition to control the autonomic nervous system or to prevent, improve or alleviate disturbances in the autonomic nervous system and which is to be ingested at an amount giving from 0.7 µg/kg to 0.09 mg/kg carnosine/body weight per ingestion.

In a second aspect the present invention provides the use of carnosine in the production of a composition to suppress the enhancement of the parasympathetic nerve system or to promote the activity of the parasympathetic nerve system and which is to be ingested at an amount giving from 0.7 µg/kg to 0.09 mg/kg carnosine/body weight per ingestion.

The composition can have an effect of improving symptoms of hypertension or hyperglycemia in which the symptoms due to disturbances in the autonomic nervous system were caused by the enhancement of the sympathetic nerve activity.

The present composition may be a pharmaceutical composition or a food or a drink.

Specific examples of such compositions include tablets containing for example 0.04 to 5 mg of carnosine per tablet, capsules containing for example 0.04 to 5 mg of carnosine per capsule, drinks and juices containing for example 0.04 to 5 mg of carnosine per container, powders or granules containing for example 0.04 to 5 mg of carnosine per pack, and foods such as cake, biscuit or bread containing for example 0.04 to 5 mg of carnosine per piece,

One or a plurality of these compositions may be ingested so that the amount of carnosine ingested is the amount having an effect of controlling the autonomic nervous system, i.e. it does not exceed 0.1 mg/kg body weight per ingestion in humans. These compositions may contain 0.04 mg or less of carnosine per tablet, container, pack, or piece. In this case, a plurality of compositions may be ingested so that the amount of carnosine ingested does not exceed 0.1 mg/kg body weight per ingestion in humans.

### Brief Explanation of the Drawings

Figure 1 shows the suppressive effect of carnosine on the efferent neural activity of the sympathetic nerve adrenal branches in rats.
Figure 2 shows the suppressive effect of carnosine on the efferent neural activity of the sympathetic nerve hepatic branches in rats.
Figure 3 shows the suppressive effect of carnosine on the efferent neural activity of the sympathetic nerve renal branches in rats.
Figure 4 shows the promoting effect of carnosine on the efferent neural activity of the vagus nerve celiac branches in rats.
Figure 5 shows the suppressive effect of intraperitoneally administered carnosine on an increase in blood glucose levels in the 2DG hyperglycemic rats.
Figure 6 shows the suppressive effect of intracranially administered carnosine on an increase in blood glucose levels in the 2DG hyperglycemic rats.
Figure 7 shows the suppressive effect of intracranially administered carnosine on an increase in blood glucose levels in the STZ diabetic rats undergoing the oral glucose tolerance test.
Figure 8 shows the suppressive effect of intraperitoneally administered carnosine on an increase in blood glucose levels in the STZ diabetic rats undergoing the oral glucose tolerance test.
Figure 9 shows the suppressive effect of the ingestion of carnosine-containing diets in the DOCA salt hypertensive rats.
Figure 10 shows the promoting effect of carnosine on the activity of the parasympathetic nerve system in humans.

The present invention will now be explained more specifically.

The effect of carnosine at a low dose on the activity of the autonomic nervous system was investigated using an experimental procedure (Nippon Rinsho 48:150-158, 1990) in rats that permits the direct measurement of the autonomic nerve activity. This experimental procedure is characterized in that the effect of carnosine on the neural activity of the autonomic nervous system controlling each organ can be individually determined. Since the autonomic nervous system is responsible for the control of blood glucose level, the control of blood pressure, the control of gastric juice secretion, the control of body temperature and the like ("The regulatory system in organisms" in Iwanami Koza "Gendai Igaku-no Kiso (Basic Modern Medicine)" Vol. 4, edited by Toshio Hagiwara and Seiichi Tarui, 1999), the direct control of the autonomic nerve activity would allow regulation of these control mechanisms in the living body.

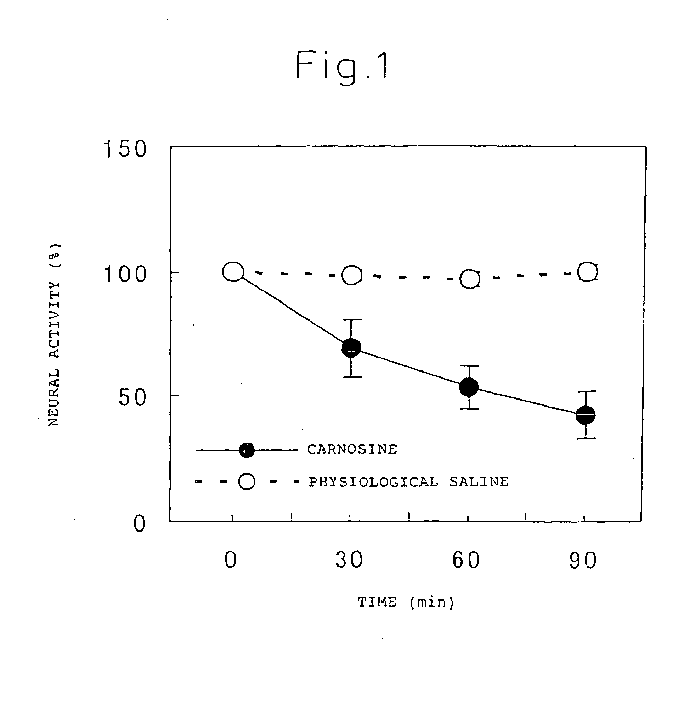

First, in the examination on the effect of carnosine at a low dose on the efferent neural activity of the sympathetic nerve adrenal branches, the intravenous administration of 100 ng per rat (0.00033 mg/kg body weight) of carnosine suppressed the efferent neural activity of the sympathetic nerve adrenal branches (Figure 1).

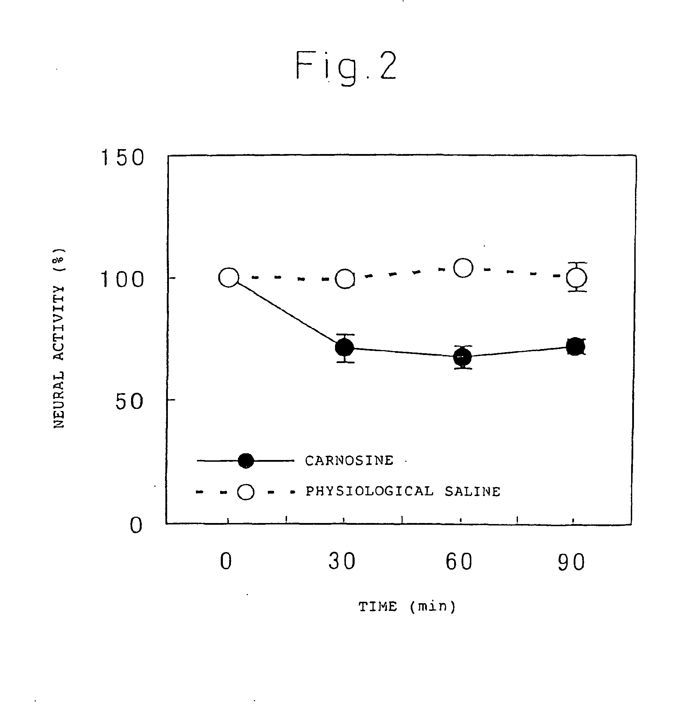

Then, in the examination on the effect of carnosine at a low dose on the efferent neural activity of the sympathetic nerve hepatic branches, the intravenous administration of 100 ng per rat (0.00033 mg/kg body weight) of carnosine suppressed the efferent neural activity of the sympathetic nerve hepatic branches (Figure 2).

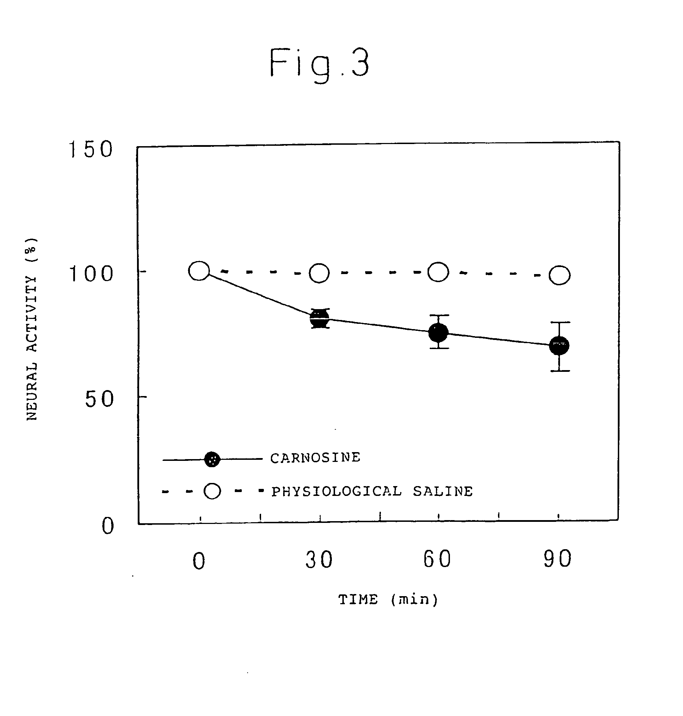

In the examination on the effect of carnosine at a low dose on the efferent neural activity of the sympathetic nerve renal branches, the intravenous administration of 1,000 ng per rat (0.0033 mg/kg body weight) of carnosine suppressed the efferent neural activity of the sympathetic nerve renal branches (Figure 3).

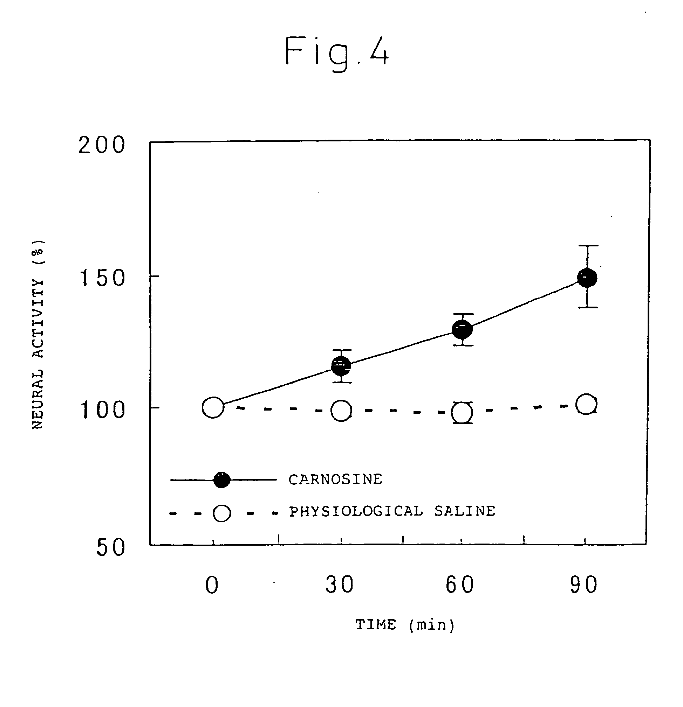

Furthermore, the effect of carnosine at a low dose on the neural activity of the vagal nerve, that is a representative parasympathetic nerve, was investigated. The vagal nerve celiac branches control most of the internal organs including the liver, the pancreas, and the gastrointestinal tracts. The intravenous administration at a low dose of 1,000 ng per rat (0.0033 mg/kg body weight) of carnosine promoted the efferent neural activity of the vagal nerve celiac branches (Figure 4).

The above results revealed that carnosine at low doses exhibited an effect of controlling the autonomic nervous system. That is, it acts directly on the sympathetic nerve system and the parasympathetic nerve system.

The controlling effect of carnosine at low doses on the autonomic nervous system was further confirmed by the following investigation.

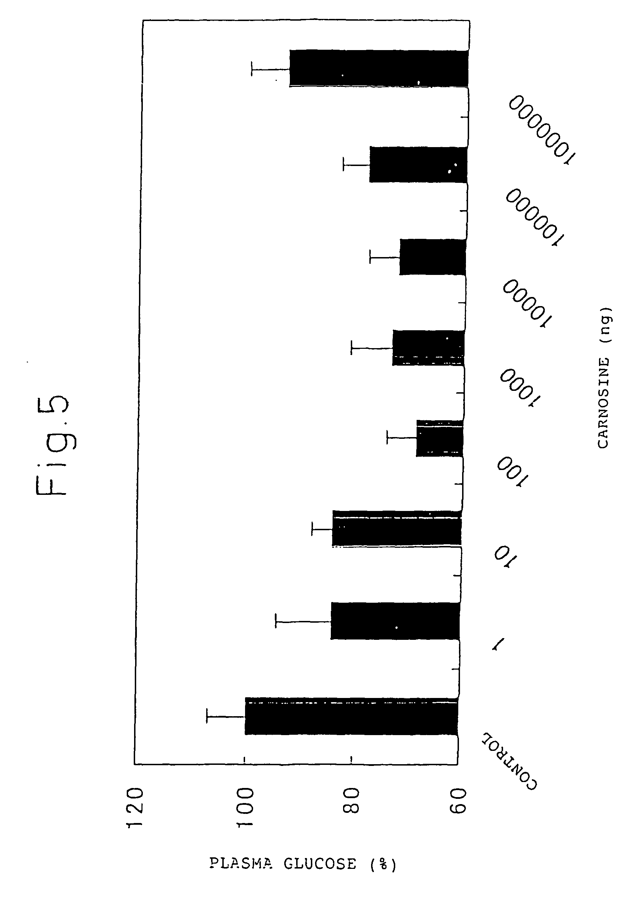

In order to investigate the effect of carnosine at low doses on improving hyperglycemia in a sympathetic nerve system-dominant state, the 2-deoxy-D-glucose (2DG) hyperglycemic rats (Brain Research 809:165-174, 1998) were used as the animal model of hyperglycemia. By intraperitoneal administration of carnosine at 10 to 100,000 ng (0.000033 mg to 0.33 mg/kg body weight) to a 2DG hyperglycemic rats, a hypoglycemic effect was noted (Figure 5). However, the intraperitoneal administration of carnosine at a high dose of 1,000,000 ng (3.3 mg/kg body weight) did not show any hypoglycemic action. This result is believed to indicate that carnosine acted on the autonomic nervous system only when ingested at low doses (10 ng to 100 µg/ingestion) resulting in decreased blood sugar, and thereby improved the hyperglycemia in a state in which the neural activity of the sympathetic nerve system is dominant.

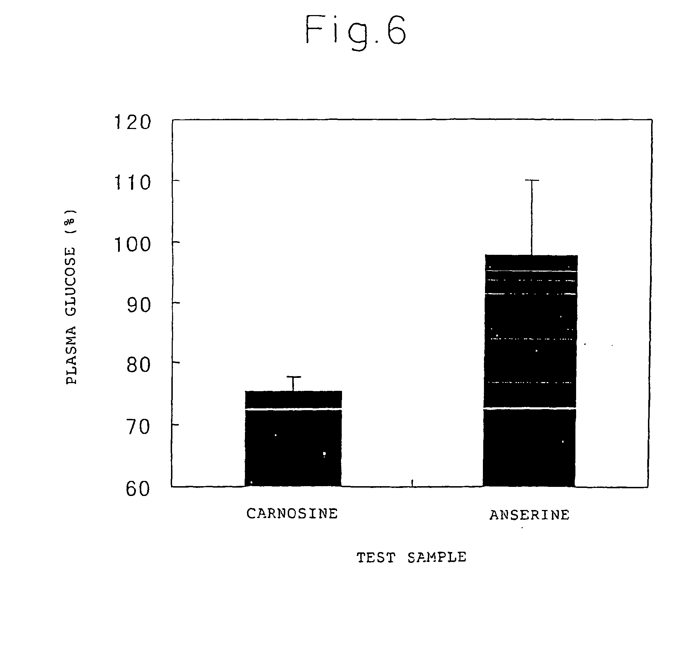

It was investigated whether this hypoglycemic effect exhibited by carnosine can be observed for anserine that has a similar structure to carnosine. As a result, no hypoglycemic effect was noted when 10 ng (0.000033 mg) of anserine was intracranially administered to the 2DG hyperglycemic rats (Figure 6). On the other hand, the hypoglycemic effect was observed when carnosine at the same dose was intracranially administered. Since the hypoglycemic effect by carnosine is believed to result from a receptor-mediated specific action by carnosine (Brain Res. 158:407-422, 1978), the above result is possibly due to the fact that anserine, although having a similar structure, cannot bind to the receptor.

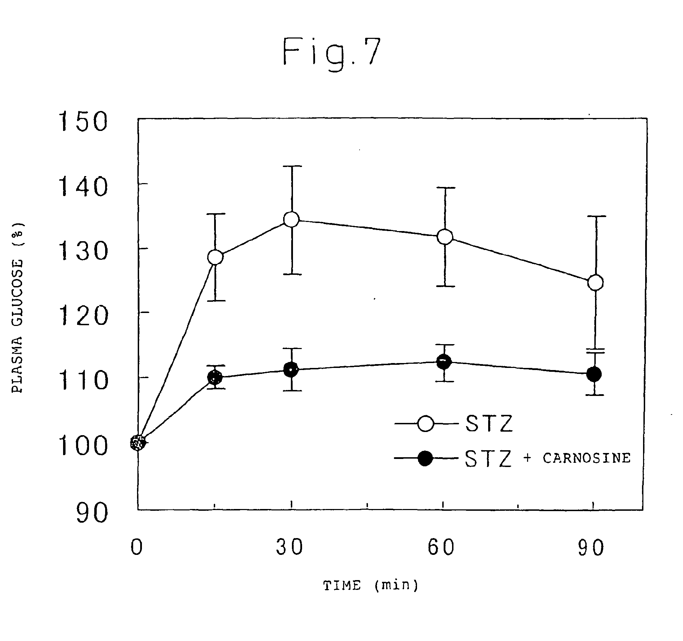

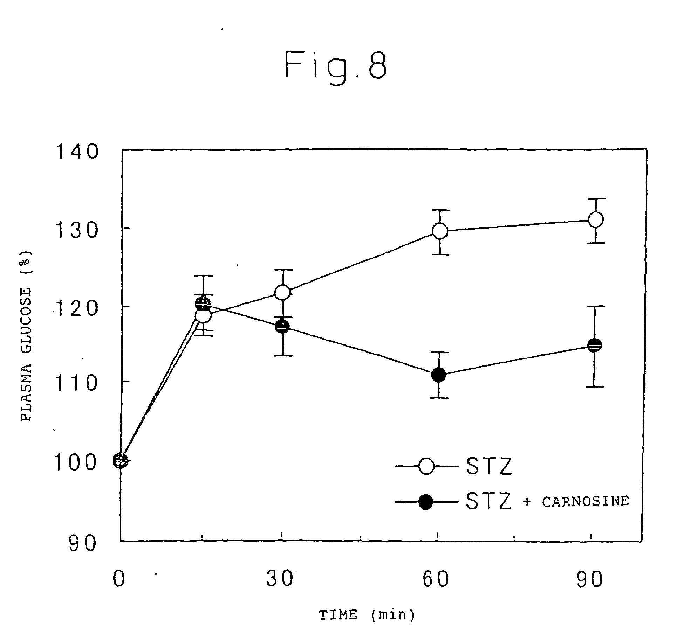

The inventors further investigated whether carnosine at a low dose acted on the autonomic nervous system and thereby exhibited a hypoglycemic effect in the streptozotocin (STZ) diabetic rats (Nippon Rinsho 56:732-737, 1998), a rat hyperglycemic model. Unlike the 2DG hyperglycemic model, this model is a diabetic model in which pancreatic beta cells have been destroyed by STZ and which develops the hyperglycemia due to the inhibition of proinsulin biosynthesis. Blood glucose levels were increased in STZ-induced diabetic rats by the oral glucose loads, and the effect of carnosine at a low dose on an increase in blood glucose levels was investigated. When 10 ng (0.000033 mg/kg body weight) of carnosine was intracranially administered (Figure 7) or 10 ng (0.000033 mg/kg body weight) of carnosine was intraperitoneally administered to a rat, the effect of restoring an increase in blood glucose levels due to the oral glucose load was confirmed (Figure 8).

In the STZ diabetic rats, carnosine concentration in the diaphragm is decreased relative to the normal rats (Metabolism 29:605-616, 1980), in addition to the insufficiency of insulin secretion, which suggests that the pathology of the hyperglycemia due to the insufficiency of insulin secretion is associated with a decrease in carnosine concentration. Thus, it is thought that the administration of carnosine caused a reduction in blood glucose levels in the hyperglycemic state as well that resulted from factors other than the enhanced activity of the sympathetic nerve system, as a result of the promoted glucose uptake in the liver due to the vagal nerve activity in the liver promoted by carnosine, and this indicated that carnosine at a low dose has an anti-diabetic effect mediated by the autonomic nervous system.

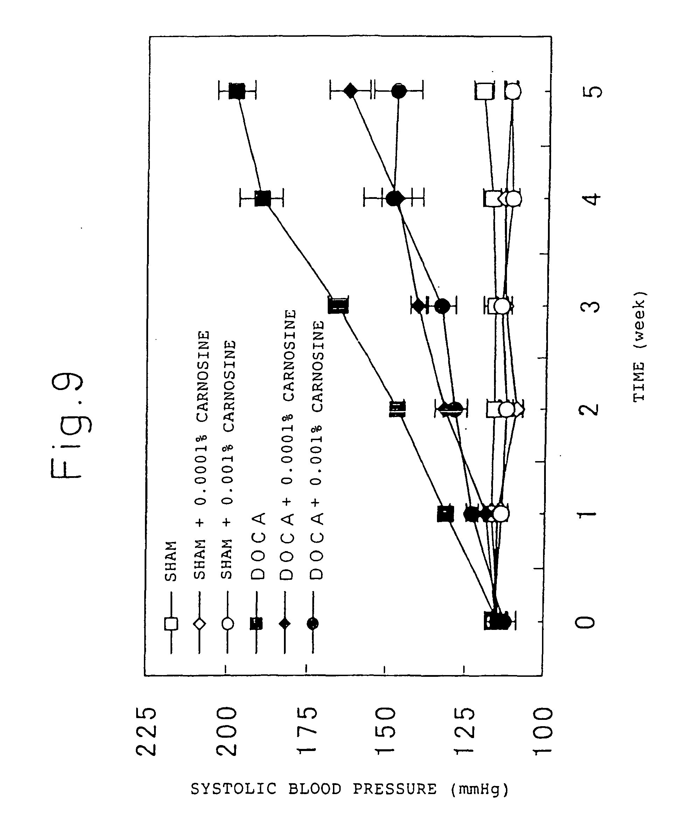

The effect of carnosine at a low dose, that acts on the autonomic nervous system and thereby improves a hypertensive state in which the sympathetic nerve activity is dominant, was investigated using the deoxycorticosterone acetate (DOCA)-salt hypertensive rats (Nippon Rinsho 58:708-712, 2000) which is an animal model of hypertension. When a diet containing 0.001% or 0.0001% carnosine was given to the DOCA-salt hypertensive rats, an hypotensive effect was noted (Figure 9).

Blood pressure is maintained at the normal level by a complex interaction between the tension of the peripheral arteries and the contractive force of the heart. As this is largely controlled by the sympathetic nerve system ("Brain and Stress" in Brain Science series Vol. 13 , edited by Tetsuo Hirano, Akira Niijima, pp. 136-137, 1995, Kyoritsu Shuppan), it was thought that carnosine at a low dose suppressed the activity of the sympathetic nerve system and hence decreased blood pressure. This result was also believed to indicate that carnosine at a low dose improved hypertension in a state in which the sympathetic nerve activity is dominant.

On the other hand, the effect on the autonomic nervous system was investigated by measuring an ECG in humans. Heart rate is controlled by the autonomic nervous system and changes with respiration and blood pressure (heart rate variation). The time sequence of heart rate variation was spectrally analyzed and the characteristic fluctuation (peaks in the spectrum) in the frequency range of about 0.1 Hz (cycle of about 10 seconds) and about 0.3 Hz (about 3 seconds) were determined (Science 213:220-222, 1981) to investigate the effect of carnosine on the activity of the autonomic nervous system.

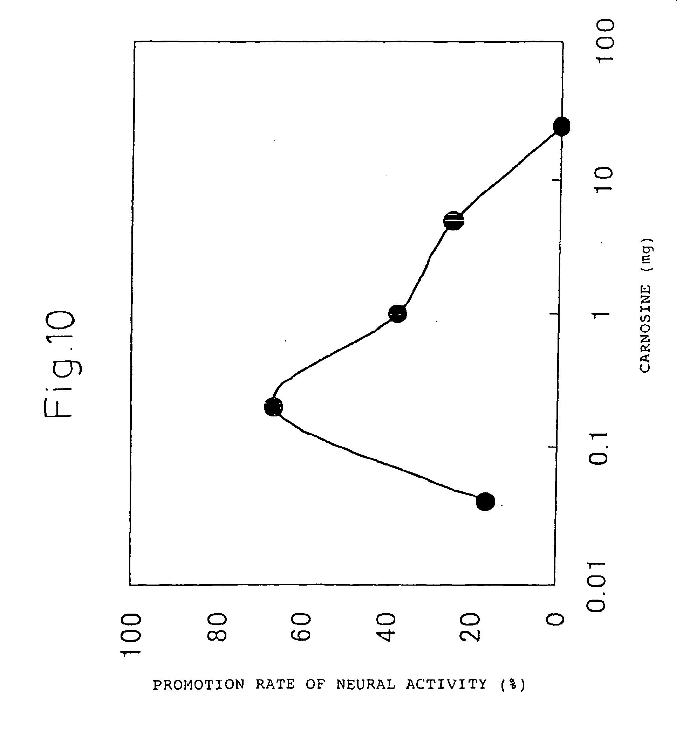

As a result, when 0.04 mg to 5 mg of carnosine per subject was ingested, the parasympathetic nerve activity of the heart was enhanced by more than two times relative to the activity before the ingestion (previous value) (Figure 10). Since the enhancement in overall activity of the autonomic nervous system was below that of the parasympathetic nerve system at this time, it indicates that carnosine at a low dose suppresses the activity of the sympathetic nerve system. The result demonstrates that the effect by carnosine of controlling the autonomic nervous system in animal experiments was reproduced in humans as well.

From the above results, the present inventors have shown that carnosine at a low dose has an effect of controlling the autonomic nervous system, that is, suppress the activity of the sympathetic nerve system, and promote the activity of the parasympathetic nerve system.

The dosage of carnosine that exhibits the effect of controlling the autonomic nervous system can be determined from the dosage of carnosine to the 2-deoxy-D-glucose (2DG) hyperglycemic rats that were used as an animal model of hyperglycemia. Thus, since the intraperitoneal administration of 10-100,000 ng of carnosine to rats caused a decrease in blood glucose levels and the oral absorptivity of carnosine in mice is 30-70% (Am. J. Physiol. 255:G143-G150, 1988), the oral administration of 33-330,000 ng of carnosine to rats should induce a controlling effect of carnosine on the autonomic nervous system in rats. Furthermore, since the body weight of the rats used in the Examples were about 300 g, the oral administration of 110 ng to 1.1 mg/kg body weight should induce a controlling effect of carnosine on the autonomic nervous system in rats. As described in the prior art, it is known that when carnosine is to be orally administered to experimental animals, the amount of 100 mg/kg body weight/day or more is required. Carnosine according to the present findings exhibits efficacy at smaller doses (one 90th to one 900,000th) of those conventionally used. According to the present findings, moreover, the oral administration of carnosine to rats at 10 mg/kg body weight/day or more does not exhibit any effect of controlling the autonomic nervous system.

For example, although the intraperitoneal administration of 10 to 100,000 ng of carnosine to rats suppressed increases in blood glucose levels in the 2DG hyperglycemic rats, the intraperitoneal administration of 10 to 100,000-fold more, or 1 mg, of carnosine did not show any effect of suppressing increases in blood glucose levels in the 2DG hyperglycemic rats (Figure 5). The dose-response curve of the hypoglycemic effect by carnosine takes a bell shape. In the study of agonists of glutamate receptors, the dose-response curves of the agonist's effects take a bell shape in many cases, and agonists are characteristically effective at extremely low concentrations (Nichiyakurishi 116:125-131, 2000).

Although glutamate receptors regulate the excitatory control of nerve cells and the release of transmitters, the signaling system of the glutamate receptors do not always act in a excitatory manner, which is believed to a reason why the dose-response curve is bell-shaped. Thus, it is thought that the signaling system of glutamate receptors activates not only one pathway but, in a concentration-dependent manner (at high concentrations), other signaling pathways. Since the presence of receptors have been reported for carnosine (Brain Res. 158:407-422, 1978), it is thought to have a bell-shaped dose-response curve similar to the glutamate receptor agonists.

On the other hand, when the doses for animals are used to estimate those for humans, the coefficient 10 that has taken into consideration for the species difference (Evaluation of Food Safety, edited by Kageaki Aihara, Mitsuru Uchiyama, Gakkai Shuppan Center, 1987), the dosage for humans will be 1/10 that for rats. Thus, in the case of the present invention, the oral dosage for humans is estimated to be 11 ng to 0.11 mg/kg body weight.

In fact, after the effect of carnosine on the autonomic nerve activity in the human heart was investigated, the enhancement in the parasympathetic nerve activity was observed when low doses of 0.04 mg to 5 mg of carnosine per subject were administered. By converting this in terms of body weight of subjects, the dosages were 0.000741 mg/kg to 0.0926 mg/kg body weight. When this was compared to the dosage of carnosine for humans estimated from the effective dosage for animals, the upper limit was consistent with the estimated oral dosage. However, the lower limit was different by nearly about 100-fold.

This is probably due to the fact that the activity of a carnosine-degrading enzyme in the blood is, in the case of humans, 2-7 µmol/hr/ml (Pediat. Res. 7:601-606, 1973), whereas no activity of carnosine-degrading enzyme is detected in the blood in the case of rats (Biochim. Biophys. Acta. 429(1):214-219, 1976). The present inventors have determined the carnosine-degrading enzyme in the plasma of rats, and found that it was 0.01-0.04 µmol/hr/ml which is about 100-fold lower than in humans. Thus, it indicates that, in the case of small amounts of carnosine, since carnosine is rapidly degraded in the human blood, about 100-fold more carnosine is required for humans than for rats. The result of dosage for humans estimated from the effective dosage of carnosine for animals can be explained from the difference in the activity of carnosine-degrading enzymes.

When carnosine according to the present proposals is used as a modulator of the autonomic nervous system, preferably, small doses are ingested during the hours not affected by meals. For example, they are preferably ingested before going to bed or between meals. Furthermore, for vegetarians who avoid ingesting meat and largely take vegetables, to ingest carnosine as the modulator of the autonomic nervous system is a preferred mode.

Carnosine is plentiful in chicken and beef. For example, 280 mg of carnosine is contained in 100 g of chicken, and 150 mg of carnosine is contained in 100 g of beef (Adv. Enzyme Regul. 30:175-194, 1990). From the content described in this article, the amount (for example 5 mg) of carnosine that promotes the parasympathetic nerve activity may be calculated to be 1.78 g of chicken. Furthermore, it is known that breast has the highest content of carnosine in chicken, and it is not practical to ingest such a small amount of chicken as to promote the activity of the parasympathetic nerve system.

Furthermore, according to an article (Am. J. Physio. 255:G143-G150, 1988), when carnosine was administered to the jejunum of mice to investigate its absorptivity, the absoptivity is more than two-fold higher when administered in combination with L-alanine than when carnosine alone is administered. When administered with β-alanine, the absorptivity becomes more than tripled, indicating that the absorptivity of carnosine varies with the type of the concomitant amino acid. Thus, as shown herein, in order to control the activity of the autonomic nervous system with carnosine at a low dose, it is essential to ingest a precise low amount of carnosine, and therefore it is not practical to ingest carnosine from the diet at amounts that can control the autonomic nerve activity.

When carnosine according to the present proposals is used as a modulator of the autonomic nervous system, not only health-beneficial foods and drinks that contain carnosine but food additives that contain carnosine are also included. When used as health-beneficial foods and drinks, it may be blended into, but not limited to, dry foods, dietary supplements, refreshing drinks, mineral waters and alcoholic drinks.

When the carnosine is used in health-beneficial foods and drinks and food additives, it may be processed as a pharmaceutical preparation. The pharmaceutical preparations may be solid or liquid, and as such there may be mentioned powders, tablets, pills, capsules, granules, suspensions, emulsions, and the like. For a pharmaceutical preparation made in the present invention, a pharmaceutically acceptable excipient may be added. As the excipients, there can be used diluents, flavoring agents, stabilizers, lubricants for suspension, binders, preservatives, disintegrants for tablets, alone or in combination.

When the carnosine is used as a therapeutic agent, the pharmaceutical preparation may be solid or liquid, and as such there may be mentioned powders, tablets, pills, capsules, suppositories, granules, liquid preparations for internal use, suspensions, emulsions, lotions, and the like. For the pharmaceutical preparation, ion, a pharmaceutically acceptable excipient may be added. As the excipients, there can be used diluents, flavoring agents, stabilizers, lubricants for suspension, binders, preservatives, disintegrants for tablet, and the like alone or in combination.

The present invention will now be explained with reference to the Examples.

### Example 1. Regulatory effect of carnosine on the rat autonomic nervous system

According to the method of Niijima (Neurobiology 3:299-307, 1995), the effect of carnosine on the autonomic nerve activity was investigated. Wistar male rats, weighing about 300 g, that had been kept in an environment of room temperature (24°C) and free access to the feed were fasted (with free access to water) 12 hours before the start of the experiment. Rats were subjected to abdominal section under urethane anesthesia (1 g/kg body weight, intraperitoneal administration), neurofilaments were isolated from central cut ends of a sympathetic nerve branch or a vagal nerve branch, and the efferent neural activity was recorded via an AC amplifier. The neural activity was observed using an oscilloscope and was recorded into magnetic tape. At the same time, the time course of the neural activity was recorded with a pen-type recorder via a rate meter with a reset time set at 5 seconds.

Carnosine (100 ng or 1,000 ng dissolved in 0.1 ml physiological saline) was intravenously administered. As the control, physiological saline (0.1 ml) was alone administered intravenously.

The effect of carnosine administration was calculated as the neural activity count (%) after carnosine administration relative to neural activity count (mean value of 10 activity counts during 5 minutes) before administration set at 100. Five rats were used for each experiment. Data were tested with ANOVA (p<0.05) and were expressed as mean +/- standard error.

### (1) The suppression of the efferent neural activity of the sympathetic nerve adrenal branches

By the intravenous administration of 100 ng of carnosine to rats, the efferent neural activity of the sympathetic nerve adrenal branches was significantly suppressed at 30 minutes (suppression rate 31.3%), 60 minutes (suppression rate 46.9%), and 90 minutes (suppression rate 58.1%) after administration (Figure 1).

### (2) The suppression of the efferent neural activity of the sympathetic nerve hepatic branches

By the intravenous administration of 100 ng of carnosine to rats, the efferent neural activity of the sympathetic nerve hepatic branches was significantly suppressed at 30 minutes (suppression rate 20.1%), 60 minutes (suppression rate 25.9%), and 90 minutes (suppression rate 32.0%) after administration (Figure 2).

### (3) The suppression of the efferent neural activity of the sympathetic nerve renal branches

By the intravenous administration of 1,000 ng of carnosine to rats, the efferent neural activity of the sympathetic nerve renal branches was significantly suppressed at 30 minutes (suppression rate 29.7%), 60 minutes (suppression rate 32.8%), and 90 minutes (suppression rate 28.0%) after administration (Figure 3).

### (4) The promotion of the efferent neural activity of the vagal nerve celiac branches

In order to study the effect on the neural activity of the vagal nerve, the efferent neural activity of the vagal nerve celiac branches was recorded. By the intravenous administration of 100 ng carnosine to rats, the efferent neural activity of the vagal nerve was significantly promoted at 30 minutes (promotion rate 15.4%), 60 minutes (promotion rate 28.6%), and 90 minutes (promotion rate 48.7%) after administration (Figure 4).

The above results indicated that, as the intravenous administration of carnosine at low doses suppressed the activity of the sympathetic nerve system and promoted the activity of the vagal nerve, carnosine at a low dose has an effect of controlling the autonomic nervous system.

### Example 2. Effect of carnosine on anti-hyperglycemia in the 2DG hyperglycemic rats

Since blood glucose levels are expected to be reduced when the activity of the autonomic nervous system is controlled by carnosine at a low dose, the effect of carnosine on anti-hyperglycemia in the 2DG hyperglycemic rats was investigated according to the method of Chun et al. (Brain Research 809:165-174, 1998). Wistar male rats (initial weight, about 250 g) were used that had been acclimated for at least 10 days to an environment in a room illuminated with an incandescent light of 80 lux on the average for 12 hours at room temperature (24 +/-1°C) with free access to the feed.

Three days before the experiment, rats under anesthesia with pentobarbital (35 mg/kg body weight, intraperitoneal administration) received the insertion of a cardiac catheter comprising a silastic tubing (Dow Corning, Midland, MI) and a polyethylene tubing (Clay Adams, Parsippany, NJ) into the right atrium and a polyethylene tubing into the right lateral cerebral ventricle (LCV). On the day of the experiment, carnosine dissolved in physiological saline was administered to the rats intracranially (0.01 ml) or intraperitoneally (0.1 ml) with the simultaneous administration into the rat LCV of 80 µmol 2-deoxy-D-glucose (2DG, 0.01 ml). The control rats received physiological saline instead of carnosine and an artificial cerebrospinal fluid in stead of 2DG, each at the same amount.

Sixty minutes after the administration of 2DG into the LCV, 300 µl of blood was taken via the cardiac catheter and the plasma level of glucose was determined. The glucose concentrations in the plasma were measured using the Fuji-Dri-chem system (Fuji Film Tokyo) according to the glucose oxidase method. Changes in plasma concentrations of glucose were expressed as a ratio (%) relative to the control value at 90 minutes after 2DG administration set at 100. Four or five rats were used in the experiments. Data were tested with ANOVA (p<0.05) and were expressed as mean +/- standard error.

### (1) The suppression of hyperglycemia by intraperitoneally administered carnosine

To the 2DG hyperglycemic rats, carnosine at 1 ng, 10 ng, 100 ng, 1,000 ng, 10,000 ng, 100,000 ng, and 1,000,000 ng was intraperitoneally administered, and changes in plasma levels of glucose were investigated. As a result, the hyperglycemia was significantly suppressed by the intraperitoneal administration of carnosine at 10 ng (suppression rate 16.0%), 100 ng (suppression rate 31.5%), 1,000 ng (suppression rate 26.8%), 10,000 ng (suppression rate 27.6%), and 100,000 ng (suppression rate 21.9%) (Figure 5).

### (2) Comparison of anserine and carnosine

Anserine, that has a similar structure to carnosine, was intracranially administered to compare the effect of anti-hyperglycemia. The intracranially administration of 10 ng carnosine to rats suppressed hyperglycemia, but the same amount of anserine did not suppress hyperglycemia (Figure 6). This result demonstrates that the effect of carnosine on anti-hyperglycemia is an unique reaction to carnosine.

### Example 3. Effect of carnosine on anti-hyperglycemia in the STZ diabetic rats undergoing the oral glucose tolerance test

Wistar male rats (initial weight, about 250 g) were used. They were housed in a room illuminated with an incandescent light of 80 luxes on the average for 12 hours with free access to feed, and adapted to the environment at least 10 days. Three days before the experiment, rats under anesthesia with pentobarbital (35 mg/kg body weight, intraperitoneal administration) received the insertion of a cardiac catheter comprising a silastic tubing (Dow Corning, Midland, MI) and a polyethylene tubing (Clay Adams, Parsippany, NJ) into the right atrium and a polyethylene tubing into the right lateral cerebral ventricle (LCV).

Then, the STZ (streptozotocin) diabetic rats were created according to the report by Portha et al. (Diabetes 30:64-69, 1981). Streptozotocin (STZ, manufactured by Sigma) dissolved in 0.05 M citric acid solution (pH 4.5) at a concentration of 2 g/ml was intraperitoneally administered at 60 mg/kg body weight. To the STZ diabetic rats thus created, an aqueous solution of glucose dissolved at 0.5 g/ml was orally administered at 1 ml (0.5 g) per animal (oral glucose load). Simultaneously with the glucose load, carnosine dissolved in physiological saline was administered to the rats intracranially (0.01 ml) or intraperitoneally (0.1 ml).

The oral administration employed a sonde, and the intracranially administration was performed via a LVC polyethylene tube and the intraperitoneal administration employed an injection syringe and a needle. The control rats received physiological saline instead of carnosine, and an artificial cerebrospinal fluid instead of 2DG, each at the same amount. Before and at 15, 30, 60, and 90 minutes after the oral administration of glucose, 300 µl of blood was taken via the cardiac catheter and the glucose concentrations in the plasma (blood glucose levels) were determined. The glucose concentration in the plasma at the time of carnosine administration was calculated as a ratio (%) relative to the plasma glucose concentration before glucose administration set at 100. Four or five rats were used in the experiment. Data were tested with ANOVA (p<0.05) and were expressed as mean +/standard error.

When 10 ng of carnosine was intracranially administered to rats, increases in blood glucose by oral glucose load was significantly suppressed from 15 to 90 minutes (Figure 7). The suppression rate was 14.5% at 15 minutes, 17.3% at 30 minutes, 14.7% at 60 minutes, and 11.4% at 90 minutes. Also, when 100 ng of carnosine was intraperitoneally administered to the rats, increases in blood glucose levels by the oral glucose load was significantly suppressed at 60 and 90 minutes (Figure 8). The suppression rate was 14.3% at 60 minutes and 12.4% at 90 minutes.

### Example 4. Anti-hypertensive effect of carnosine in DOCA-salt hypertensive rats

Sprague-Dawley male rats (6-week old) were used. The animals were anesthetized by the intraperitoneal administration of pentobarbital at 40 mg/kg body weight, and the right flank was dissected to remove the right kidney. After the surgical operation and a one week recovery period, the animals were divided into the pseudosurgery (sham) group and the deoxycorticosterone acetate (DOCA) salt group. Each group was further divided into the normal diet group, the 0.001% carnosine-containing diet group, and the 0.0001% carnosine-containing group.

The rats of the DOCA-salt group received the subcutaneous injection of DOCA suspended in corn oil at 15 mg/kg body weight twice per week, and tap water supplemented with 1% sodium chloride was given as the drinking water. The sham group received neither DOCA nor sodium chloride. The systolic blood pressure was monitored once per week for five weeks using the tail cuff and the pneumatic pulse transducer (BP-98A, Softron). The experiment employed 9 rats in the DOCA group and 6 rats in the sham group. Data were tested with ANOVA (p<0.05) and were expressed as mean +/standard error.

The systolic blood pressure in the rats of the DOCA-salt-normal diet group increased from week one and blood pressure increased up to 200 mmHg at week 5. On the other hand, in the rats of the DOCA-salt-0.001% carnosine-containing diet group and the DOCA-salt-0.0001% carnosine-containing diet group, increases in blood pressure were significantly suppressed from week one as compared to the rats in the DOCA-salt-normal diet group (Figure 9). The DOCA-salt-0.0001% carnosine-containing diet group showed a suppression rate of 9.6% at week one, 10.2% at week two, 15.0% at week three, 22.1% at week four, and 17.8% at week five. The DOCA-salt-0.001% carnosine-containing diet group showed a suppression rate of 6.2% at week one, 12.4% at week two, 19.6% at week three, 21.5% at week four, and 25.5% at week five. The above result indicates that carnosine-containing diets significantly suppress increases in blood pressure in a state in which the activity of the sympathetic nerve system is dominant.

### Example 5. Regulatory effect of carnosine on the autonomic nervous system in humans

The resting ECG of the subjects was measured for 5 minutes, and ECG was measured for 5 minutes at 30 minutes and 60 minutes after drinking the test sample, respectively. In order to avoid influences by changes in respiration, ECG was measured when the subjects were forced to respire at 15 respirations per minute. The intervals between R-waves (R-R intervals) of the ECG were determined, and were subjected to a power spectral analysis according to the program prepared by Moritani et al. (J. Sport Med. Sci. 7:31-39, 1993). For cycle components, in conformity to the classification criteria of the Cardiology society in Europe and the USA (Circulation 93:1043-1065, 1996), heart rate variation was divided into the low frequency (0.03-0.15 Hz) and the high frequency (0.15-0.4 Hz).

Carnosine was weighed out on a chartula at 0.04 mg, 0.2 mg, 1 mg, 5 mg, and 25 mg/capita. For 0.2 mg, one mg of a 1:4 mixture of carnosine and starch powder (1/5 dilution) was weighed out. For 0.04 mg, one mg of a 1:4 mixture of carnosine and starch powder that further mixed with the starch powder at 1:4 (1/25 dilution) was weighed out. Carnosine was taken by licking the one weighed out on chartula.

There were 8 subjects, 21-51 years old (33.0 +/-11.95 years old) and weighing 54-73.5 kg (64.3 +/- 7.01 kg), and among them, the experiments of the 0.04 mg and the 0.2 mg were performed on six subjects, the experiments of the 1 mg and the 5 mg were performed on 8 subjects, and the experiment of the 25 mg was performed on 5 subjects. The evaluation of neural activity was expressed as a ratio of humans that exhibited more than 200% of the previous value of the parasympathetic nerve activity.

The influence on the autonomic nerve activity was observed in one of the six subjects in the 0.04 mg carnosine ingestion experiment (the ratio of those who exhibited promotion: 16.7%, the promotion ratio of the parasympathetic nerve activity: 249%, the promotion ratio of the overall autonomic nerve activity: 195%); in four of the six subjects in the 0.2 mg ingestion experiment (the ratio of those who exhibited promotion: 66.7%, the mean promotion ratio of the parasympathetic nerve activity: 436%, the mean promotion ratio of the overall autonomic nerve activity: 329%); in three of the eight subjects in the 1 mg ingestion experiment (the ratio of those who exhibited promotion: 37.5%, the mean promotion ratio of the parasympathetic nerve activity: 259%, the maen promotion ratio of the overall autonomic nerve activity: 190%); in two of the eight subjects in the 5 mg ingestion experiment (the ratio of those who exhibited promotion: 25.0%, the mean promotion ratio of the parasympathetic nerve activity: 472%, the mean promotion ratio of the overall autonomic nerve activity: 410%); in 0 of the eight subjects in the 25 mg ingestion experiment (the ratio of those who exhibited promotion: 0%).

The above result indicates that carnosine promotes the activity of the parasympathetic nerve system in humans as well. The dosage was 0.04 mg/dose to 5 mg/dose (0.000741 mg/kg to 0.0926 mg/kg). The ratio of the subjects who exhibited the promotion of the parasympathetic nerve activity of 200% or more of the previous value is shown in Figure 10. On the other hand, the overall autonomic nerve activity was below the promotion ratio of the parasympathetic nerve activity in all concentrations. This indicates that carnosine suppresses the activity of the sympathetic nerve system.

| (Formulation Example 1) Tablets | |
|---|---|
| | (% by weight) |
| Carnosine | 2 |
| Lactose | 83 |
| Heavy magnesium oxide | 15 |

were uniformly mixed to formulate tablets of 100 mg per tablet.

| (Formulation Example 2) Powders and granules | |
|---|---|
| | (% by weight) |
| Carnosine | 2 |
| Lactose | 70 |
| Starch | 28 |

were uniformly mixed to formulate powders or granules.

| (Formulation Example 3) Capsules | |
|---|---|
| | (% by weight) |
| Gelatin | 70 |
| Glycerin | 22.9 |
| Methyl parahydroxybenzoate | 0.15 |
| Propyl parahydroxybenzoate | 0.35 |
| Water | Appropriate amount |
| Total 100% | |

The composition shown in Formulation Example 1 was filled into a soft capsule coat comprising the above ingredients to obtain soft capsules of 100 mg per granule.

| (Formulation Example 4) Drink preparation | | |
|---|---|---|
| Taste: | DL-sodium tartarate | 0.1 g |
| | Succinic acid | 0.009 g |
| Sweet taste: liquid sugar | | 800 g |
| Acid taste: citric acid | | 12 g |
| Vitamin: vitamin C | | 10 g |
| Carnosine | | 0.2 g |
| Vitamin E | | 30 g |
| Cyclodextrin | | 5 g |
| Flavor | | 15 ml |
| Potassium chloride | | 1 g |
| Magnesium sulfate | | 0.5 g |

The above ingredients were mixed and water was added to make 10 liters. This drink preparation is taken at about 100 ml per dose.

### Industrial Applicability

The ingestion of carnosine at only a low dose permits the suppression of the efferent neural activity of the sympathetic nerve system and the promotion of the efferent neural activity of the parasympathetic nerve system. Such an effect of controlling the autonomic nervous system is extremely useful in the prevention and alleviation of an unbalanced autonomic nervous system.

## Claims

1. The use of carnosine in the production of a composition to control the autonomic nervous system or to prevent, improve or alleviate disturbances in the autonomic nervous system and which is to be ingested at an amount giving from 0.7 µg/kg to 0.09 mg/kg carnosine/body weight per ingestion.

2. The use of carnosine in the production of a composition to suppress the enhancement of the parasympathetic nerve system or to promote the activity of the parasympathetic nerve system and which is to be ingested at an amount giving from 0.7 µg/kg to 0.09 mg/kg carnosine/body weight per ingestion.

3. Use according to claim 2 in which the composition is for the treatment of symptoms of hypertension or high blood sugar

4. Use according to any one of the preceding claims in which the composition is a pharmaceutical composition.

5. Use according to claim 4 in which the composition is in the form of tablets or capsules.

6. Use according to claim 5 in which the capsules or tablets contain from 0.04 to 5 mg carnosine each.

7. Use according to any one of claims 1 to 3 in which the composition is in the form of a drink or juice.

8. Use according to claim 7 in which the drink or juice is in a container, containing from 0.04 to 5 mg carnosine per container.

9. Use according to any one of claims 1 to 3 in which the composition is in the form of powder or granules in packs each containing from 0.04 to 5 mg carnosine.

10. Use according to any one of claims 1 to 3 in which the composition is prepared in the form of a food containing from 0.04 to 5 mg carnosine per piece.

## Patentansprüche

1. Verwendung von Carnosin bei der Herstellung einer Zusammensetzung zur Kontrolle des autonomen Nervensystems oder zur Vorbeugung, Verbesserung oder Linderung von Störungen im autonomen Nervensystem und die in einer Menge eingenommen werden soll, die 0,7 *µ*g/kg bis 0,09 mg/kg Carnosin/Körpergewicht pro Einnahme ergibt.

2. Verwendung von Carnosin bei der Herstellung einer Zusammensetzung zur Unterdrückung der Steigerung des parasympathischen Nervensystems oder zur Förderung der Aktivität des parasympathischen Nervensystems und die in einer Menge eingenommen werden soll, die 0,7 µg/kg bis 0,09 mg/kg Carnosin/Körpergewicht pro Einnahme ergibt.

3. Verwendung gemäß Anspruch 2, bei der die Zusammensetzung zur Behandlung von Symptomen des Bluthochdrucks oder des hohen Blutzuckers dient.

4. Verwendung gemäß mindestens einem der vorhergehenden Ansprüche, bei der die Zusammensetzung eine pharmazeutische Zusammensetzung ist.

5. Verwendung gemäß Anspruch 4, bei der die Zusammensetzung in Form von Tabletten oder Kapseln vorliegt.

6. Verwendung gemäß Anspruch 5, bei der die Kapseln oder Tabletten jeweils 0,04 bis 5 mg Carnosin enthalten.

7. Verwendung gemäß mindestens einem der Ansprüche 1 bis 3, bei der die Zusammensetzung in Form eines Getränks oder Safts vorliegt.

8. Verwendung gemäß Anspruch 7, bei der das Getränk oder der Saft in einem Behälter vorliegt, der von 0,04 bis 5 mg Carnosin pro Behälter enthält.

9. Verwendung gemäß mindestens einem der Ansprüche 1 bis 3, bei die Zusammensetzung in Form von Pulver oder Granalien in Packungen, die jeweils 0,04 bis 5 mg Carnosin enthalten, vorliegt.

10. Verwendung gemäß mindestens einem der Ansprüche 1 bis 3, bei der die Zusammensetzung in Form eines Nahrungsmittels, das 0,04 bis 5 mg Carnosin pro Stück enthält, hergestellt ist.

## Revendications

1. Utilisation de carnosine dans la production d'une composition pour contrôler le système nerveux autonome ou pour prévenir, améliorer ou soulager les troubles du système nerveux autonome, et qui doit être ingérée en une quantité de 0,7 µg/kg à 0,09 mg/kg de carnosine/poids corporel à chaque ingestion.

2. Utilisation de carnosine dans la production d'une composition pour inhiber le renforcement du système nerveux parasympathique ou pour favoriser l'activité du système nerveux parasympathique, qui doit être ingérée en une quantité de 0,7 µg/kg à 0,09 mg/kg de carnosine/poids corporel à chaque ingestion.

3. Utilisation selon la revendication 2 dans laquelle la composition est destinée au traitement de symptômes d'hypertension ou d'hyperglycémie.

4. Utilisation selon l'une quelconque des revendications précédentes dans laquelle la composition est une composition pharmaceutique.

5. Utilisation selon la revendication 4 dans laquelle la composition est sous la forme de comprimés ou de gélules.

6. Utilisation selon la revendication 5 dans laquelle les gélules ou les comprimés contiennent de 0,04 à 5 mg de carnosine chacun.

7. Utilisation selon l'une quelconque des revendications 1 à 3 dans laquelle la composition est sous la forme d'une boisson ou d'un jus.

8. Utilisation selon la revendication 7 dans laquelle la boisson ou le jus sont dans un récipient contenant de 0,04 à 5 mg de carnosine par récipient.

9. Utilisation selon l'une quelconque des revendications 1 à 3 dans laquelle la composition est sous la forme d'une poudre ou de granules dans des emballages contenant chacun de 0,04 à 5 mg de carnosine.

10. Utilisation selon l'une quelconque des revendications 1 à 3 dans laquelle la composition est préparée sous la forme d'un aliment contenant de 0,04 à 5 mg de carnosine par morceau.
